# EUROPEAN PATENT SPECIFICATION

(11) **EP 2 488 183 B1**
(45) Date of publication and mention of the grant of the patent: **28.08.2013**
(21) Application number: 10823620.9
(22) Date of filing: 15.10.2010
(51) Int. Cl.: A61K 31/52, A61K 31/522, A61K 31/704, A61K 45/06, A61P 35/00, A61P 29/00

(54) **Composition for inhibiting TGF-beta comprising imidazopurine derivatives**
Zusammensetzung zur Hemmung von TGF-beta mit Imidazopurinderivaten
Composition d'inhibition de TGF-beta comprenant des dérivés d'imidazopurines

(30) Priority: 15.10.2009 KR 20090098416
(43) Date of publication of application: 22.08.2012
(73) Proprietor: Korea Institute of Radiological & Medical Sciences, Seoul 139-709 (KR)
(72) Inventor: SONG, Jie Young, Seoul 122-737 (KR); YUN, Yeon Sook, Seoul 135-837 (KR); AHN, Ji Yeon, Seoul 136-777 (KR); JUNG, In Sung, Seoul 131-781 (KR); UM, Hong Duck, Seoul 110-550 (KR); PARK, Sarah, Seoul 135-776 (KR); LIM, Min Jin, Seoul 139-240 (KR); KIM, Mi Hyoung, Seoul 139-240 (KR); LEE, Sae Lo Oom, Seoul 140-869 (KR)
(74) Representative: Johnstone, Helen Margaret
(86) International application number: PCT/KR2010/007071
(87) International publication number: WO 2011/046381

(56) References cited:
- WO-A1-98/13350
- WO-A1-2004/018430
- WO-A1-2005/080377
- US-A1- 2002 103 211
- PARK S ET AL: "IM-412 inhibits transforming growth factor beta-induced fibroblast differentiation in human lung fibroblast cells", BIOCHEMICAL AND BIOPHYSICAL RESEARCH COMMUNICATIONS, ACADEMIC PRESS INC. ORLANDO, FL, US, vol. 399, no. 2, 20 August 2010 (2010-08-20), pages 268-273, XP027425316, ISSN: 0006-291X, DOI: 10.1016/J.BBRC.2010.07.067 [retrieved on 2010-08-20]
- DATABASE REGISTRY [Online] CHEMICAL ABSTRACTS SERVICE, COLUMBUS, OHIO, US; 2 February 2007 (2007-02-02), XP002676995, Database accession no. 919012-48-9
- DATABASE REGISTRY [Online] CHEMICAL ABSTRACTS SERVICE, COLUMBUS, OHIO, US; 2 February 2007 (2007-02-02), XP002676996, Database accession no. 919012-40-1

## Description

### Technical Field

The present invention provides a compound of imidazo[2,1-f]purine-2,4(3H,8H)-dion or a pharmaceutically acceptable salt thereof for use in treating or preventing cancer, inflammation or fibrosis.

The present invention is obtained from a research performed as a government-funded project of the Ministry of Education, Science and Technology.

### [Project Name: Development of Chemical Genomics System for Development of Radiation Therapy Controller]

### Background Art

Transforming growth factor-β (TGF-β) is a multifunctional cytokine that controls many cellular biological processes including cell proliferation, apoptosis, cell differentiation, immunity, transformation, and extracellular matrix accumulation and is expressed in most cells. If TGF-βis over-expressed, extracellular matrix (ECM) is excessively accumulated in organs, leading to fibrosis/sclerosis. It was reported that over-expression of TGF-β occurs in most diseases related to fibrosis/sclerosis, for example, diabetic nephropathy, or fibrosis of liver, lungs, or pancreas (EP 0998936A1; Pancreas, 2005, 30(3), e71-9).

In addition, TGF-β functions as a tumor suppressor at an initial stage of a tumor but thereafter, functions as a tumor promoter. It is well known that TGF-β is excessively formed from a tumor, inhibits natural killer (NK) cells or macrophages which directly control an initial immune response and degrades an antigen-presenting function of a tumor, thereby inhibiting the activity of T lymphocytes and promoting tumor growth, infiltration, and metastasis. In addition, it was also reported that TGF-β is indirectly involved in angiogenesis by up-regulating generation of a vascular edothelial growth factor (VEGF) (Am. J. Respir Crit Care Med. 2002, 165, 88-94; Cancer Res. 2003, 63, 1083-1092). Accordingly, TGF-β may promote tumor growth, infiltration, and metastases by neoangiogenesis or inhibition of immune activity. It was shown that when radioactive therapy or an anti-cancer agent such as doxorubicin is applied in a breast cancer transplanted mouse model, the level of TGF-β is substantially increased and metastasis to a lung is increased, but when an anti TGF-β antibody is administered, such changes are reduced, which proves that combination therapy including anti-cancer agents or radiation therapy is effective in inhibiting generation or activity of TGF-β(J Clin Invest. 1993, 92, 2569-2576; Curr. cancer Drug targets 2006, 75, 565-578). In addition, according to a recent research, TGF-β inhibition induces inhibition of ataxia telangientasia-mutated (ATM) gene which repairs a chromosome double cleavage generated due to various stimulations such as radiation, an anti-cancer agent, a ultraviolet ray, or active oxygen species, thereby increasing sensitivity of tumor cells (Kirshner J. et al. Cancer Research, 2006, 66(22), 10861-10869). Accordingly, it is deemed that when a TGF-β inhibitor is administered in combination with radiation and an anti-cancer agent, its therapeutic efficiency would be increased.

It is reported that TGF-β inhibitors are effective in treating diseases such as fibrosis, cirrhosis of liver, Crohn s disease, dermatosclerosis, cardiomyopathy, rheumatic arthritis, chronic graft-versus-host disease, or cancer, which are caused by over-expression of TGF-β. For example, an antibody against TGF-β is effective in treating glomerulonephritis (Nature, 1990, 346, 371-374), scars in neurons or skin (Eur. J. Neurosci. 1994, 6, 355-363; Lancet, 1992, 339, 213-214), lung fibrosis (Thorax 1993, 48, 959-966), radiation-induced fibrosis(US Patent No. 5,616,561), myelofibrosis, bums, Dupuytren's contracture, gastric ulcers, and rheumatic arthritis (J. Exp. Medicine, 1993, 177, 225-230). Accordingly, there is a continuing need to develop medicines for controlling reactions of TGF-β that plays a critical role in pathogenesis of various diseases. In response to such a need, we have studied to develop materials that show anti-inflammation, anti-fibrosis, and anti-cancer adjuvant effects via inhibition of TGF-β activity and completed the present invention. US 2002/0103211 describes tricyclic compounds for the treatment or prevention of the diseases affected by cytokine intracellular signalling.

### Disclosure of Invention

### Technical Problem

The present disclosure provides imidazopurine derivatives capable of inhibiting transforming growth factor-β(TGF-β).

The present invention provides a composition for preventing or treating cancer, inflammation or fibrosis, comprising imidazopurine derivatives capable of inhibiting TGF-β.

The present invention also provides a composition for enhancing an activity of an anti-cancer agent, including imidazopurine derivatives capable of inhibiting TGF-β.

### Solution to Problem

According to an aspect of the present invention, there is provided a compound of imidazo[2,1-f]purine-2,4(3H,8H)-dion of Formula A below or a pharmaceutically acceptable salt thereof for use in treating or preventing cancer, inflammation or fibrosis: 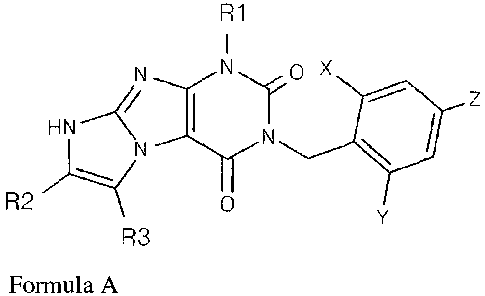 wherein
X, Y, and Z are each independently hydrogen, or halo, and
R1, R2 and R3 are each independently hydrogen, a C₁-C₄ alkyl, or a C₁-C₄ alkyl that is selectively substituted with one or more substituents selected from the group consisting of hydroxy, halo, a C₁-C₄ alkyl, and a C₁-C₄ haloalkyl,
wherein the halo is fluoro, chloro, bromo, or iodo.
TGF-β is a mutifunctional cytokine that mediates inflammations and fibrosis and functions as a tumor promoter in a tumor. Accordingly, an agent that inhibits TGF-β activity may be used to prevent or treat various diseases through anti-cancer, anti-inflammation and anti-fibrosis effects. It was shown that when a cell in which fibrosis progressed due to TGF-β was treated with the compound of Formula A, the fibrosis was inhibited. In addition, it was found that when an inflamed cell was treated with the compound of Formula A, a pro-inflammatory cytokine was significantly reduced.

According to an embodiment of the present invention, the imidazo[2,1-f]purine-2,4(3H,8H)-dion compound may be 3-(2-chlorobenzyl)-1,7-dimethyl-1H-imidazo[2,1-f]purine-2,4(3H,8H)-dion of Formula I below.

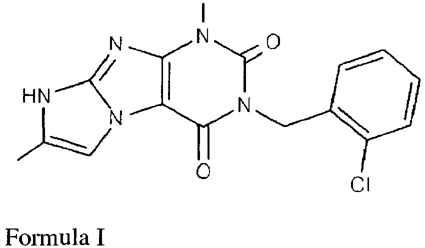

According to another embodiment of the present invention, the imidazo[2,1-f]purine-2,4(3H,8H)-dion compound may be
3-(2-chloro-6-fluorobenzyl)-1,6,7-trimethyl-1H-imidazo[2,1-f]purine-2,4(3H,8H)-dion of Formula II below.

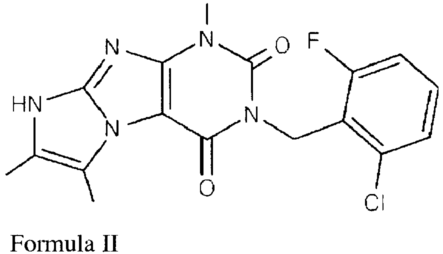

A composition according to an embodiment of the present invention may be used to prevent and treat a disease caused by over-expression of TGF-β selected from fibrosis or cirrhosis of various organs, multiple sclerosis, cardiomyopathy, Crohn's disease, dermatosclerosis, glomerulonephritis, postoperative adhesion, keloid and hypertrophic scars, proliferative vitreoretinopathy, coneal injury, chronic graft-versus-host disease (GVHD), neoangiogenesis, tumors, burns, and rheumatic arthritis.

According to an embodiment of the present invention, the composition may be used to prevent or treat cancer, inflammations, or fibrosis.

According to an embodiment of the present invention, the fibrosis may be a fibrosis of lungs, liver, or pancreas.

The present invention also provides a composition for aiding an anti-cancer agent, comprising imidazo[2,1-f]purine-2,4(3H,8H)-dion of Formula A below or or pharmaceutically acceptable salt thereof, 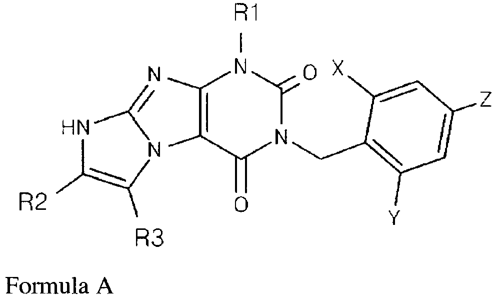 wherein
X, Y, and Z is each independently hydrogen or halo, and
R1, R2 and R3 are each independently hydrogen, a C₁-C₄ alkyl, or, a C₁-C₄alkyl that is selectively substituted with one or more substituents selected from the group consisting of hydroxy, halo, a C₁-C₄ alkyl, and a C₁-C₄ haloalkyl,
wherein the halo is fluoro, chloro, bromo, or iodo.

It was found that inhibition of TGF-β activity enhanced the sensitivity of cancer cells to radiations or anti-cancer agents. When the compound of Formula A was administered before radiation or administration of an anti-cancer agent such as doxorubicin, an anti-cancer effect, that is, apoptosis of cancer cells was enhanced.

The term "C₁-C₄alkyl" used herein refers to a linear or branched C₁-C₄alkyl, and may be, for example, methyl, ethyl, n-propyl, iso-propyl, n-butyl, iso-butyl, tert-butyl, secbutyl, or isomers thereof.

The term "C₁-C₄haloalkyl" used herein refers to a linear or branched C₁-C₄alkyl as defined above, with one or more hydrogen atoms thereof substituted with one or more halogens, in the same or different manner. Examples of the C₁-C₄haloalkyl include chloromethyl, fluoropropyl, fluoromethyl, difluoromethyl, trichloromethyl, 2,2,2-trifluoroethyl, pentafluoroethyl, bromobutyl, trifluoromethyl, iodoethyl, and isomers thereof.

The term "composition for aiding an anti-cancer agent" used herein refers to a composition that synergistically enhances an effect of an anti-cancer agent by increasing the sensitivity of a cancer cell to the anti-cancer agent, when the composition is administered in combination with the anti-cancer agent.

The term "anti-cancer agent" used herein refers to an agent that inhibits proliferation of cancer cells or killing cancer cells, and includes, but is not limited to, radioactive rays, such as gamma ray, or a chemotherapeutic agent, such as cisplatin, doxorubicin, or vinblastin.

According to an embodiment of the present invention, the anti-cancer agent may be selected from the group consisting of radiation, cisplatin, doxorubicin, vinblastin, fluorouracil, irinotecan, paclitaxel, vinorelbine, and gemcitabin.

According to an embodiment of the present invention, the imidazo[2,1-f]purine-2,4(3H,8H)-dion compound may be 3-(2-chlorobenzyl)-1,7-dimethyl-1H-imidazo[2,1-f]purine-2,4(3H,8H)-dion of Formula I below.

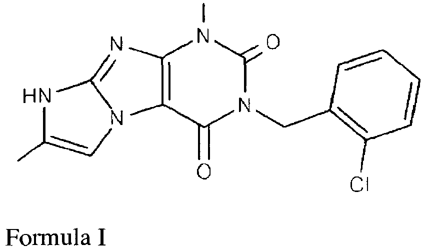

According to an embodiment of the present invention, the imidazo[2,1-f]purine-2,4(3H,8H)-dion compound may be 3-(2-chloro-6-fluorobenzyl)-1,6,7-trimethyl-1H-imidazo[2,1-f]purine-2,4(3H,8H)-dion of Formula II below.

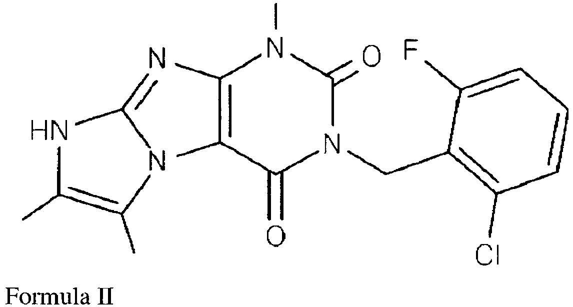

The present invention will be described in further detail with reference to the following examples.

### Advantageous Effects of Invention

A composition according to the present invention may be used to prevent or treat a disease caused by over-expression of TGF-β such as cancer, inflammations or fibrosis, by inhibiting TGF-β activity, and enhance an anti-cancer effect by increasing the sensitivity of cancer cells to an anti-cancer agent.

### Brief Description of Drawings

FIG. 1 is a diagram of a system for screening a transforming growth factor-β (TGF-β) inhibitor, in which a vector including a PAI promoter that reacts with TGF-β and a SMAD binding site is stably inserted into HEK 293 cells and then luciferase activity is measured;
FIG. 2 shows that luciferase activity is increased by stimulation of TGF-β in the screening system according to an embodiment of the present invention, thereby confirming effectiveness of the searching system. In FIG. 2, pGL2 refers to a cell strain that is transformed by a vector including only a gene encoding luciferase, 3TP-pGL2 refers to a cell strain that is transformed by a vector including a TGF-β response element (TRE), and TGF-b refers to a 3TP-pGL2 cell strain that is stimulated by TGF-β at an indicated concentration;
FIGS. 3a and 3b show that compounds I and II concentration-dependently inhibit TGF-β signaling, respectively;
FIGS. 4a and 4b show that compounds I and II do not show toxicity with respect to HEK 293 cells, respectively;
FIGS. 5a and 5b show the effect of compounds I and II on the proliferation of human hepatic stellate cells, respectively;
FIGS. 6a and 6b show that compounds I and II effectively inhibit fibrosis caused by an increase in α-smooth muscle actin and fibronectin, an extracellular matrix, in a human hepatic stellate cell, respectively;
FIGS. 7a and 7b and FIGS. 7c and 7d respectively show that compound I inhibits fibrosis of a human normal lung fibrocyte induced by TGF-β and radiation;
FIGS. 8a and 8b show that compounds I and II have an anti-inflammatory effect by inhibiting a pro-inflammatory cytokine activity of a macrophage stimulated by LPS; and
FIGS. 9a and 9b and FIGS. 9c and 9d respectively show that when compounds I and II are applied in combination with doxorubicin as an anti-cancer agent, there is a synergy effect for apoptosis of a lung cancer cell (H1299) and a brain cancer cell (U87-MG). In FIGS. 9a, 9b, 9c and 9d, C, Com I, Com II and Doxo respectively represent control group, compound I, compound II, and doxorubicin.

### Mode for the Invention

### Example 1. Screening of TGF-β inhibitor

In order to screen an agent for inhibiting TGF-β cells stably transformed by luciferase-expressing plasmids were used to measure an effect on TGF-β activity.

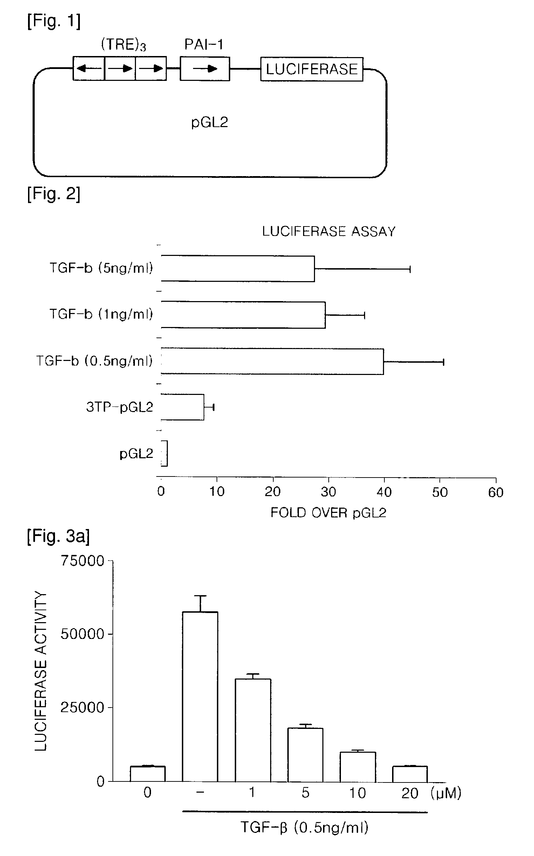

A plasmid with a Smad binding site and a platelet activator inhibitor (PAI) promoter, obtained from professor Carlos Arteaga at the University of Vanderbilt in US, was inserted into pGL2-Null reporter plasmid (Promega, U.S.A.). FIG. 1 shows a schematic structure of 3TP-pGL2 plasmid including TGF-β response element (TRE), which is the Smad binding site, a PAI-1 promoter, and a luciferase gene. The plasmid was used to transform human embryonic kidney (HEK) 293 cells according to the manufacturer s specification, using Lipofectamine 2000 (Invitrogen, Calsbad, CA, USA), thereby constructing a system for screening an effect on TGF-β activity by measuring luciferase activity. The activity of luciferase was measured using a Dual Luciferase Assay kit (Promega, Medison, WI, USA). The HEK 293 cells used were purchased from the American Type Culture Collection (ATCC), and incubated under 5% CO₂ at a temperature of 37°C in a Dulbecco's modified Eagle's medium (DMEM) supplemented with 10% fetal bovine serum (FBS), 100 units/ml of penicillin, and 100 µg/ml of streptomycin.

The HEK 293 cells transformed were inoculated into a 96-well plate at 20,000 cells/ well in triplicates and then incubated under the condition as described above for 24 hours. Then, TGF-β was added thereto at concentrations of 0.5, 1, and 5 ng/ml and after 15-hour incubation, luciferase activity was measured. After the treatment with TGF-β, the luciferase activity was increased 30 to 40 times greater than that before the treatment with TGF-β and the optimal efficacy was observed when the concentration of TGF-β was 0.5 ng/ml. FIG. 2 shows that the luciferase activity is increased by the treatment with TGF-β.

By using the screening system as described above, a TGF-β inhibition effect was tested on 8,000 compounds in a compound library. Each of the compounds was added to the 96-well plate to which the transformed HEK 293 cells were inoculated in such a way that the final concentration of each compound was 10 µM, and after two-hour incubation, 0.5 ng/ml of TGF-β was added thereto. Then, the luciferase activity was measured as described above. As a result, a low molecular weight compound having an excellent TGF-β inhibition efficacy was selected.

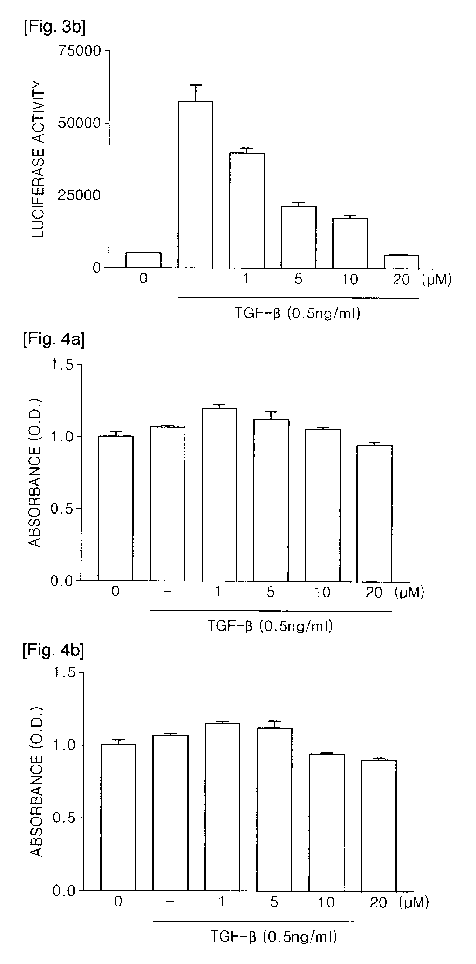

FIGS. 3a and 3b show that compounds I and II inhibit TGF-β activity in a concentration-dependent way, respectively. In order to confirm that the luciferase activity is inhibited due to TGF-β inhibitor, rather than apoptosis, a CCK-8 survival rate assay kit (Dojindo, Japan) was used and an absorbance was measured at a wavelength of 450 nm by using a spectrophotometer (Labsystems, U.S.A.) to count the number of cells. The shown values are average values obtained from three independent experiments. FIGS. 4a and 4b shows that no significant decrease in the cell count is observed in the cytotoxicity test of compounds I and II using the CCK-8 assay.

### Example 2. Suppression of Differentiation from Hepatic Stellate Cells to Hepatic Myofibrocytes, by Imidazopurine Derivatives

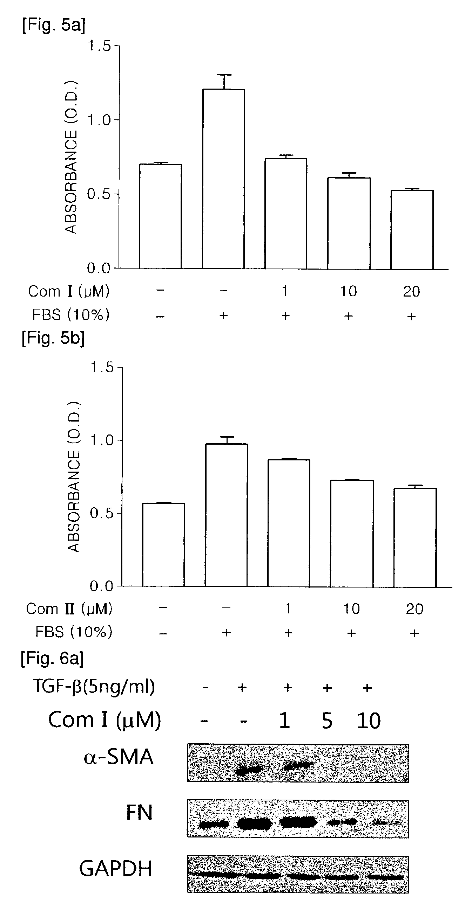

This experiment was performed to identify whether compounds I and II, which proved to effectively inhibit TGF-β activity in Example 1, inhibit fibrosis of hepatic stellate cells. HSC-T6 cells, a stable cell line made by professor S.L. Friedman at Mount Sinai in U.S. A., were obtained and used as hepatic stellate cells (HSC), which are employed mainly as a subject for liver fibrosis. HSC-T6 cells were c under 5% CO₂ at a temperature of 37°C in a DMEM supplemented with 10% FBS, 100 units/ml of penicillin, and 100 µg/ml of streptomycin. In order to identify whether compounds I and II directly affect proliferation of hepatic stellate cells, the incubation was performed in a serum-free medium for 24 hours and then the culture was treated with 1, 10, and 20 µM of the compounds I and II, 2 hours prior to addition of serum thereto. Subsequently, under the condition described above, the incubation was further performed for 48 hours and then a CCK-8 assay kit was used to measure cell viability. Absorbance was measured at a wavelength of 450 nm by using a spectrophotometer (Labsystems, U.S.A.) to count the number of cells. As illustrated in FIG. 5a, in the serum-free incubation condition, hepatic stellate cells existed in a resting phase, and when a serum was added, hepatic stellate cells rapidly proliferated and then, when treated with compound I, the proliferation of hepatic stellate cells was almost completely blocked. FIG. 5b shows the results obtained when compound II was emplyoyed under the same condition as the treatment condition for compound I. Referring to FIG. 5b, at 1, 10 and 20 µM of compound II, the proliferation of hepatic stellate cells was concentration-dependently inhibited by 12, 25, and 30%, respectively.

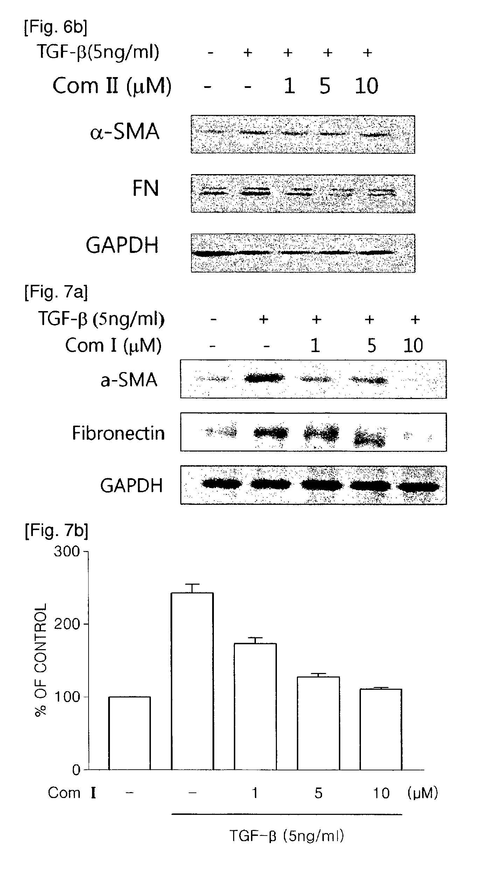

In addition, levels of α-smooth muscle actin (α-SMA) and extracellular matrix material fibronectin, which are typically used as a fibrosis molecular marker to identify fibrosis of hepatic stellate cells, were measured by western blotting. Two hours before the treatment with TGF-β(5 ng/ml), each of compounds I and II was added at a varying concentration (0, 1, 5 and 10 µM) to the HSC-T6 cells incubated under the conditions described above, and then, the HSC-T6 cells were incubated under 5% CO₂, and at a temperature of 37°C. After 24-hour incubation, the HSC-T6 cells were dissolved in a RIPA buffer solution (50 mM Tris-Cl (pH7.4), 1% NP-40, 150 mM NaCl, 1 mM EDTA) including a protease inhibitor (1 mM PMSF, 1 M/mL aprotinin, 1g/mL leupeptin, and 1 mM Na₃VO₄) to prepare cytoplasm fractions, and 4-12% gradient SDS-PAGE (Invitrogen) was performed on the cytoplasmic fractions to separate proteins. The proteins separated on the SDS-PAGE gel were transferred to a nitrocellulose membrane (BioRad, Hercules, CA, USA) and the membrane was blocked with 5% skim milk. Then, the membrane was treated with an anti-α-SMA antibody (Sigma, U.S.A.), an anti-fibronectin antibody (Santa Cruz, U.S.A.), and an anti-GAPDH antibody (Ab Frontier, Korea). Then, the membrane was reacted with a secondary antibody cojugated to horseradish peroxidase at a constant temperature, and then visualized using an ECL kit (Santa Cruz Biotechnology, Inc.). FIGS. 6a and 6b show that compounds I and II inhibit fibrosis caused by an increase in α-SMA and fibronectin in human hepatic stellate cells. Due to the treatment with TGF-β, α-SMA and fibronectin, which are fibrosis markers, were substantially increased, and due to the compound I, expression of α-SMA and fibronectin were concentration-dependently reduced. In addition, the compound II significantly reduced fibrosis markers, although the reduction was not as remarkable as that caused by compound I.

### Example 3. Suppression of Fibrosis in Human lung fibrocyte by imidazopurine derivatives

This experiment was performed to identify whether compounds I and II, which proved to effectively inhibit TGF-β activity in Example 1, inhibit fibrosis in human lung fibrocytes.

### (1) Inhibition of fibrosis caused by TGF-β

IMR-90 cells, human normal lung fibrocytes, were purchased from American Type Culture Collection (ATCC, U.S.A.), and were incubated under 5% CO₂ and at a temperature of 37°C for 24 hours in a modified Eagle's medium (MEM) supplemented with 10% FBS serum, 100 units/ml of penicillin, and 100 µg/ml of streptomycin, 1 mM sodium pyruvate, and 0.1 mM non-essential amino acids. Then, two hours before treatment with TGF-β (5ng/ml), compound I was added to the cell culture at a varying concentration (0, 1, 5 and 10 µM) and then, the IMR-90 cells were incubated for 24 hours. Then, as described in Example 2, western blotting was performed to identify inhibition of differentiation of lung fibrocytes by compound I based on expression levels of α-SMA and fibronectin, which are fibrosis marker molecules. As illustrated in FIG. 7a, compound I concentration-dependently decreased fibrosis in the human lung fibrocytes. Also, in extracellular matrix, the amount of collagen, which is one of important materials regarding fibrosis, was measured according to the specification of the manufacturer by using a collagen Sircol assay kit (Biocolor, England). Referring to FIG. 7b, when TGF-β was treated, the amount of collagen was 2.4 times greater than that obtained without TGF-β treatment, and compound I concentration-dependently reduced the amount of collagen.

### (2) Inhibition of fibrosis caused by radiation

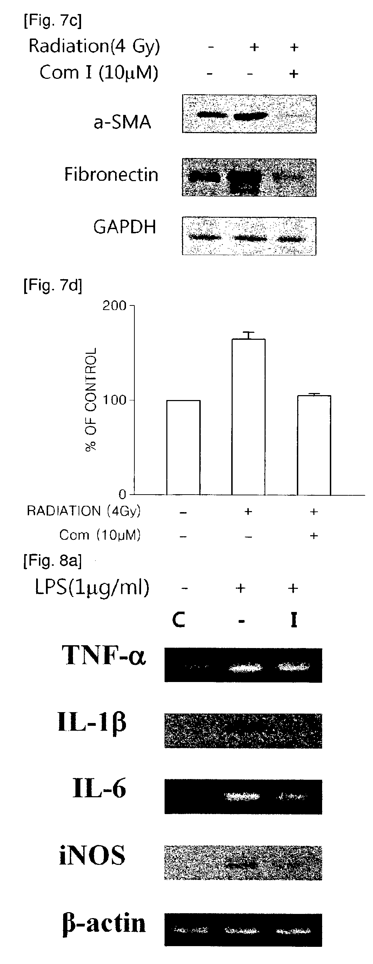

In order to identify whether compound I inhibits radiation-induced fibrosis, instead of external stimulation by TGF-β radiation that can internally engogenously increase TGF-β was employed to induce fibrosis. IMR-90 cells were incubated as described in (1) above and the IMR-90 cells were inoculated to a 6 cm plate containing a modified Eagle's medium (MEM) supplemented with 10% FBS serum, 100 units/ml of penicillin, and 100 µg/ml of streptomycin, 1 mM sodium pyruvate, and 0.1 mM non-essential amino acids. Then, compound I at a concentration of 10 µM was added thereto and the IMR-90 cells were incubated for 2 hours, and then, 4 Gy radiation was applied thereto. 48 hours later, the IMR-90 cells were collected. Then, fibrosis makers as described in Example 2 were identified from the IMR-90 cells by western blotting. As illustrated in FIGS. 7c and 7d, compound I significantly reduced expressions of α-SMA and fibronectin, and when radiation was applied, the amount of collagen was increased 1.7 times greater than that obtained without radiation. In addition, when the cells were treated with compound I, the amount of collagen was reduced to a level similar to that in the no-treatment control group.

### Example 4. Anti-inflammation Effect of Imidazopurine Derivatives on Macrophage Stimulated by LPS

A macrophage is a front-line immune cell that is responsible for an initial immune response to various antigens and causes a strong inflammation reaction. RAW 264.7 cell, a mouse macrophage strain, was purchased from American Type Culture Collection (ATCC, U.S.A.), and incubated under 5% CO₂ and at a temperature of 37°C in a Dulbecco's modified Eagle's medium (DMEM) supplemented with 10% FBS serum, 100 units/ml of penicillin, and 100 µg/ml of streptomycin. 10 mM of compound I or II was added to the incubated cells and then, two hours later, the cells were treated with 1 µg/ml lipopolysaccharide (LPS), which is a cell wall component of a gram negative bacteria and incubated further for 6 hours. Then, the cells were collected and the total RNA thereof was isolated using TRIzol and quantified. Out of the total RNA, 1 µg of RNA was used to synthesize cDNA using a reverse transcriptase. Then, a polymerase chain reaction (PCR) was performed using the cDNA as a template to identify expression levels of cytokines. In detail, PCR was performed in the presence of a DNA polymerase using the cDNA as a template to amplify IL-1β, IL-6, and TNF-α which are pro-inflammatory cytokines, iNOS, nitric oxide synthase, and β-actin used as a control group with a cycle consisting of 30 seconds at a temperature of 94°C, 45 seconds at a temperature of 55°C, and 45 seconds at a temperature of 72°C repeated 30 times. Primers set forth in SEQ ID. NOs: 1 through 10 shown in Table 1 were used to amplify IL-1β, IL-6, TNF-α, iNOS and β-actin, respectively.
Table 1

**[Table 1]**

| Primer sequences used for RT-PCR | | |
|---|---|---|
| primer | | sequence |
| IL-1 | forward | 5'-TGAAGGGCTGCTTCCAAACCTTTGACC-3' (SEQ ID. NO:1) |
| | reverse | 5'-TGTCCATTGAGGTGGAGAGCTTTCAGC-3'(SEQ ID. NO:2) |
| IL-6 | forward | 5'-TTCACAGAGGATACCACT-3'(SEQ ID. NO:3) |
| | reverse | 5'-TAGCCACTCCTTCTGTGA-3'(SEQ ID. NO:4) |
| TNF-α | forward | 5'-CAGGCAGGTTCTGTCCCTTTCA-3'(SEQ ID. NO:5) |
| | reverse | 5'-CACTTGGTGGTTTGCTACGACG-3'(SEQ ID. NO:6) |
| iNOS | forward | 5'-CCTTGTTCAGCTACGCCTTC-3'(SEQ ID. NO:7) |
| | reverse | 5'-CTGAGGGCTCTGTTGAGGTC-3'(SEQ ID. NO:8) |
| β-actin | forward | 5'-AGGCTGTGCTGTCCCTGTATGC-3'(SEQ ID. NO:9) |
| | reverse | 5'-ACCCAAGAAGGAAGGCTGGAAA-3'(SEQ ID. NO:10) |

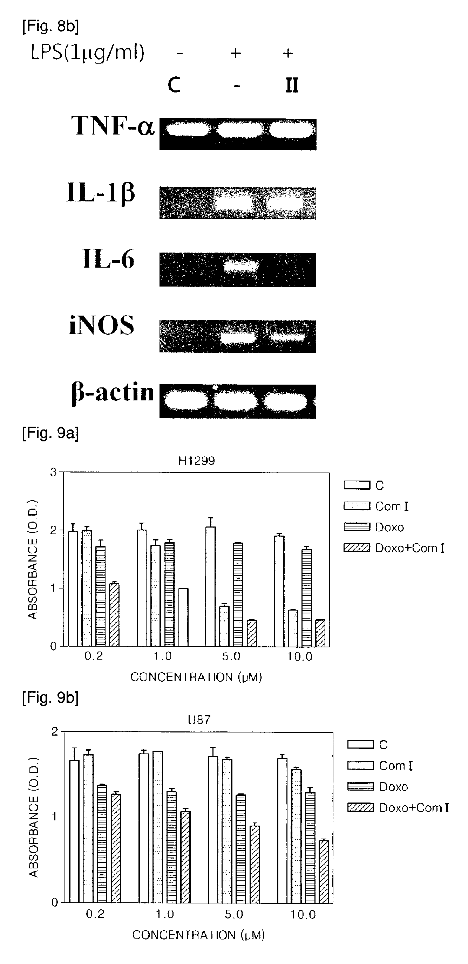

The amplification product of PCR was subjected to electrophoresis on 1% agarose gel in which ethidium bromide (dye) was present so as to measure mRNA expression levels of the cytokines. FIGS. 8a and 8b show changes in the amounts of the pro-inflammatory cytokines caused by compounds I and II, respectively. When the cells were treated with LPS, the pro-inflammatory cytokines were substantially increased, and when treated with compound I, IL-1 and iNOS were significantly inhibited, and when treated with compound II, IL-6 and iNOS were distinctively reduced. In addition, compounds I and II both slightly reduced expression of TNF-α.

### Example 5. Synergistic Anti-cancer Effect by Co-administration of Imidazopurine Derivative and Anti-cancer Agent

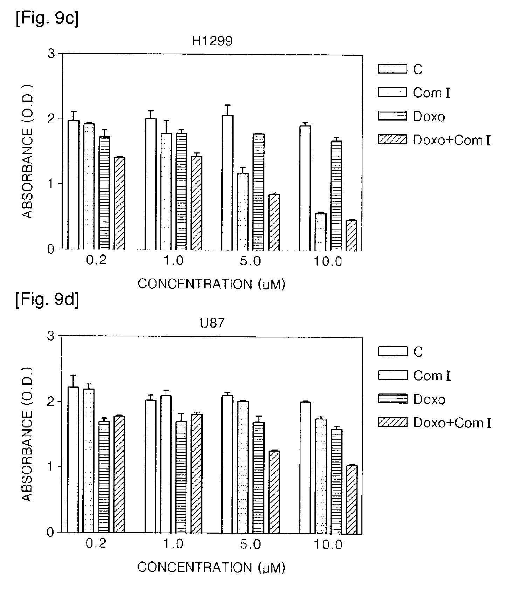

TGF-βis known to have contrasting effects related to a tumor. That is, at an initial stage, TGF-β suppresses tumor growth, but at a latter stage, promotes metastasis or infiltration of a tumor. Thus, apoptosis of tumor cell lines caused by compound I and II, and apoptosis of tumor cell lines caused by compound I or II in combination with doxorubicin, an anti-cancer agent, were measured. H1299, a lung cancer cell strain and U87-MG, a brain cancer cell strain were purchased from American Type Culture Collection (ATCC), and a concentration of doxorubicin used was 0.05 µM at which doxorubicin did not induce apoptosis by itself. The tumor cells were treated with compound I or II at the concentrations shown in FIG. 9, and 2 hours later, doxorubicin was added thereto. Then, the cells were incubated for 48 hours under the conditions described above and then, quantified using a CCK-8 viability assay kit (Dojindo, Japan). As illustrated in FIG. 9, when compound I or II was used alone at a concentration of 5 µM or more, apoptosis occurred in H1299, a lung cancer cell strain (FIGS. 9a and 9c) but did not occur in U87-MG, a brain cancer cell strain (FIGS. 9b and 9d). However, when compound I or II was used together with doxorubicin, compounds I and II showed additive or synergistic apoptosis effects on both of the tumor cells. This result shows that when an anti-cancer agent is used, compounds I and II are able to be used as an adjuvant for enhancing an efficacy of the anti-cancer agent.

### Example 6. Synergistic Anti-cancer Effect by Co-administration of Imidazopurine Derivative and Radiation

H1299, a cell strain of lung cancer, which is generally treated by radiation therapy, was treated with radiation in combination with compound I or II, and apoptosis thereof was measured. Compound I or II was added to H1299 cells inoculated to a 6 cm cell incubation plate as described in Example 3, and two hours later, 6 Gy of radioactive rays were irradiated thereto, and 48 hours later, apoptosis was measured by Annexin-PI staining and FACS assay. As shown in Table 2 below, compared to when compound I or II or radiation was used alone, when compound I or II was used together with radiation, a synergistic effect on apoptosis occurred. This result shows that when radiation therapy is employed, compounds I and II can be used as an effective adjuvant.
Table 2

**[Table 2]**

| Apoptosis induced when treated compound I or II alone or in combination with radiation | |
|---|---|
| Treatment | Apoptosis (%, average±SEM) |
| H1299cell | 2.34±0.11 |
| H1299cell + radiation (6 Gy) | 26.50±1.59 |
| H1299cell + compound I (5 µM) | 20.86±1.03 |
| 1299cell + radiation (6 Gy) + compound I (5 µM) | 73.42±1.84 |
| H1299cell + compound II (5 µM) | 14.79±0.78 |
| H1299cell + radiation (6 Gy) + compound II (5 µM) | 55.91±5.58 |

### Sequence Listing Free Text

The sequences of SEQ ID NOs: 1-10 are listed in the Sequence List enclosed herewith.
## Claims

1. A compound of imidazo[2,1-f]purine-2,4(3H,8H)-dion of Formula A below or a pharmaceutically acceptable salt thereof for use in treating or preventing a disease caused by over-expression of TGF-β selected from cancer, inflammation, and fibrosis**:** 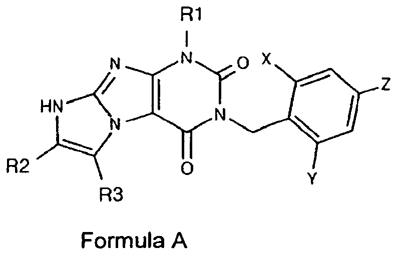 wherein
X, Y, and Z are each independently hydrogen, or halo, and
R1, R2 and R3 are each independently hydrogen, a C₁-C₄ alkyl, or a C₁-C₄ alkyl that is selectively substituted with one or more substituents selected from the group consisting of hydroxy, halo, a C₁-C₄ alkyl, and a C₁-C₄ haloalkyl,
wherein the halo is fluoro, chloro, bromo, or iodo.

2. The compound for use as in claim 1, wherein the imidazo[2,1-f]purine-2,4(3H,8H)-dion compound is 3-(2-chlorobenzyl)-1,7-dimethyl-1H-imidazo[2,1-f]purine-2,4(3H,8H)-dion.

3. The compound for use as in claim 1, wherein the imidazo[2,1-f]purine-2,4(3H,8H)-dion compound is 3-(2-chloro-6-fluorobenzyl)-1,6,7-trimethyl-1H-imidazo[2,1-f]purine-2,4(3H,8H)-dion.

4. The compound of any of claims 1 to 3 for use in treating or preventing a disease caused by over-expression of TGF-β selected from multiple sclerosis, cardiomyopathy, Crohn's disease, dermatosclerosis, glomerulonephritis, postoperative adhesion, keloid and hypertrophic scars, proliferative vitreoretinopathy, corneal injury, chronic graft-versus-host disease (GVHD), neoangiogenesis, burns, tumors and rheumatic arthritis.

5. The compound of any of claims 1 to 3, for use in enhancing the effect of an anti-cancer agent.

6. The compound for use as in claim 5, wherein the anti-cancer agent is selected from the group comprising radiation, cisplatin, doxorubicin, vinblastin, fluorouracil, irinotecan, paclitaxel, vinorelbine, and gemcitabin.

## Patentansprüche

1. Imidazo[2,1-f]purin-2,4(3H,8H)-dion-Verbindung der Formel A, unten, oder ein pharmazeutisch akzeptables Salz davon zur Verwendung bei der Behandlung oder Prävention einer durch Überexpression von TGF-β verursachten Krankheit ausgewählt aus Krebs, Entzündung und Fibrose: 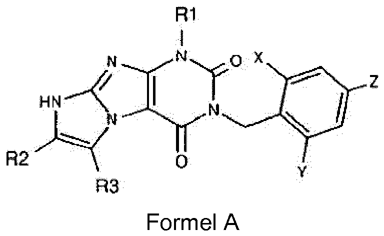 , wobei
X, Y und Z unabhängig voneinander Wasserstoff oder Halogen sind und
R1, R2 und R3 unabhängig voneinander Wasserstoff, ein C₁-C₄-Alkyl oder ein C₁-C₄-Alkyl sind, das selektiv mit einem oder mehreren Substituenten ausgewählt aus der Gruppe bestehend aus Hydroxy, Halogen, einem C₁-C₄-Alkyl und einem C₁-C₄-Halogenalkyl substituiert ist,
wobei das Halogen Fluor, Chlor, Brom oder Jod ist.

2. Verbindung zur Verwendung wie in Anspruch 1, wobei die Imidazo[2,1-f]purin-2,4(3H,8H)-dion-Verbindung 3-(2-Chlorbenzyl)-1,7-dimethyl-1H-imidazo[2,1-f]purin-2,4(3H,8H)-dion ist.

3. Verbindung zur Verwendung wie in Anspruch 1, wobei die Imidazo[2,1-f]purin-2,4(3H,8H)-dion-Verbindung 3-(2-Chlor-6-fluorbenzyl)-1,6,7-trimethyl-1H-imidazo[2,1-f]purin-2,4(3H,8H)-dion ist.

4. Verbindung nach einem der Ansprüche 1 bis 3 zur Verwendung bei der Behandlung oder Prävention einer durch Überexpression von TGF-β verursachten Krankheit ausgewählt aus multipler Sklerose, Kardiomyopathie, Crohn-Krankheit, Dermatosklerose, Glumerulonephritis, postoperativer Adhäsion, keloiden oder hypertrophen Narben, proliferativer Vitreoretinopathie, Hornhautverletzung, chronischer Transplantal-Wirt-Reaktion (graft-versus-host desease = GVHD), Neoangiogenese, Verbrennungen, Tumoren und rheumatischer Arthritis.

5. Verbindung nach einem der Ansprüche 1 bis 3 zur Verwendung beim Verbessern des Effekts eines Antikrebsmittels.

6. Verbindung zur Verwendung wie in Anspruch 5, wobei das Antikrebsmittel ausgewählt ist aus der Gruppe, die Bestrahlung, Cisplatin, Doxorubicin, Vinblastin, Fluoruracil, Irenotecan, Paclitaxel, Vinorelbin und Gemcitabin aufweist.

## Revendications

1. Composé d'imidazo[2,1-f]purine-2,4(3H,8H)-dione de formule A ci-dessous ou sel pharmaceutiquement acceptable de celui-ci, pour une utilisation dans le traitement ou la prévention d'une maladie provoquée par une surexpression de TGF-β choisie parmi un cancer, une inflammation et une fibrome : 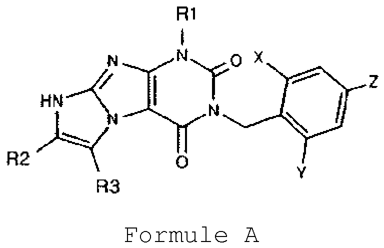 dans laquelle
X, Y et Z sont chacun indépendamment un hydrogène ou un halogéno, et
R1, R2 et R3 sont chacun indépendamment un hydrogène, un alkyle en C₁ à C₄, ou un alkyle en C₁ à C₄ qui est sélectivement substitué par un ou plusieurs substituants choisis dans le groupe consistant en un hydroxy, un halogéno, un alkyle en C₁ à C₄ et un halogénoalkyle en C₁ à C₄,
dans lequel l'halogéno est un fluoro, chloro, bromo ou iodo.

2. Composé pour une utilisation selon la revendication 1, dans lequel le composé d'imidazo[2,1-f]purine-2,4(3H,8H)-dione est la 3-(2-chlorobenzyl)-1,7-diméthyl-1H-imidazo[2,1-f]purine-2,4(3H,8H)-dione.

3. Composé pour une utilisation selon la revendication 1, dans lequel le composé d'imidazo[2,1-f]purine-2,4(3H,8H)-dione est la 3-(2-chloro-6-fluorobenzyl)-1,6,7-triméthyl-1H-imidazo[2,1-f]purine-2,4(3H,8H)-dione.

4. Composé selon l'une quelconque des revendications 1 à 3, pour une utilisation dans le traitement ou la prévention d'une maladie provoquée par une surexpression de TGF-β choisie parmi la sclérose en plaques, une cardiomyopathie, la maladie de Crohn, la dermatosclérose, la glomérulonéphrite, une adhérence post-opératoire, les cicatrices chéloïdes et hypertrophiques, la vitréorétinopathie proliférante, une lésion cornéenne, la maladie du greffon contre l'hôte (GVH), la néoangiogenèse, les brûlures, les tumeurs et la polyarthrite rhumatoïde.

5. Composé selon l'une quelconque des revendications 1 à 3, pour une utilisation dans le renforcement de l'effet d'un agent anticancéreux.

6. Composé pour une utilisation selon la revendication 5, dans lequel l'agent anticancéreux est choisi dans le groupe comprenant un rayonnement, le cisplatine, la doxorubicine, la vinblastine, le fluorouracile, l'irinotécan, le paclitaxel, la vinorelbine et la gemcitabine.


<110> Korea Institute of Radiological & Medical Sciences
<120> Composition for inhibititing TGF-beta comprising imidazopurine derivatives
<130> LEEBC/P51226EP
<140> EP10823620.9
   <141> 2010-10-15
<150> KR-10-2009-0098416
   <151> 2009-10-15
<160> 10
<170> KopatentIn 1.71
<210> 1
   <211> 27
   <212> DNA
   <213> Artificial Sequence
<220>
   <223> forward primer for IL-1B
<400> 1
   tgaagggctg cttccaaacc tttgacc 27
<210> 2
   <211> 27
   <212> DNA
   <213> Artificial Sequence
<220>
   <223> reverse primer for IL-IB
<400> 2
   tgtccattga ggtggagagc tttcagc 27
<210> 3
   <211> 18
   <212> DNA
   <213> Artificial Sequence
<220>
   <223> forward primer for IL-6
<400> 3
   ttcacagagg ataccact 18
<210> 4
   <211> 18
   <212> DNA
   <213> Artificial Sequence
<220>
   <223> reverse primer for IL-6
<400> 4
   tagccactcc ttctgtga 18
<210> 5
   <211> 22
   <212> DNA
   <213> Artificial Sequence
<220>
   <223> forward primer for TNF-alpha
<400> 5
   caggcaggtt ctgtcccttt ca 22
<210> 6
   <211> 22
   <212> DNA
   <213> Artificial Sequence
<220>
   <223> reverse primer for TNF-alpha
<400> 6
   cacttggtgg tttgctacga cg 22
<210> 7
   <211> 20
   <212> DNA
   <213> Artificial Sequence
<220>
   <223> forward primer for iNOS
<400> 7
   ccttgttcag ctacgccttc 20
<210> 8
   <211> 20
   <212> DNA
   <213> Artificial Sequence
<220>
   <223> reverse primer for iNOS
<400> 8
   ctgagggctc tgttgaggtc 20
<210> 9
   <211> 22
   <212> DNA
   <213> Artificial Sequence
<220>
   <223> forward primer for beta-actin
<400> 9
   aggctgtgct gtccctgtat gc 22
<210> 10
   <211> 22
   <212> DNA
   <213> Artificial Sequence
<220>
   <223> reverse primer for beta-actin
<400> 10
   acccaagaag gaaggctgga aa 22